# EUROPEAN PATENT SPECIFICATION

(11) **EP 1 011 494 B1**
(45) Date of publication and mention of the grant of the patent: **03.01.2007**
(21) Application number: 98910188.6
(22) Date of filing: 05.03.1998
(51) Int. Cl.: A61B 17/285, A61B 17/32, A61B 17/125

(54) **ELECTROTHERMAL DEVICE FOR SEALING AND JOINING OR CUTTING TISSUE**
ELEKTROTHERMISCHES GERÄT ZUM VERSCHWEISSEN UND ZUSAMMENFÜGEN ODER ZUM SCHNEIDEN VON GEWEBE
DISPOSITIF ELECTROTHERMIQUE PERMETTANT DE SCELLER ET D'ASSEMBLER OU DE COUPER DES TISSUS

(30) Priority: 05.03.1997 US 38589 P
(43) Date of publication of application: 28.06.2000
(73) Proprietor: The Trustees of Columbia University in the City of New York, New York, New York 10027 (US)
(72) Inventor: TREAT, Michael, R., New York, NY 10032 (US)
(74) Representative: Goddar, Heinz J.
(86) International application number: PCT/US1998/004348
(87) International publication number: WO 1998/038935

(56) References cited:
- US-A- 3 625 202
- US-A- 4 662 068
- US-A- 5 171 255
- US-A- 5 300 065
- US-A- 5 336 221
- US-A- 5 401 273
- US-A- 5 403 312
- US-A- 5 496 317
- US-A- 5 599 350

## Description

### FIELD OF THE INVENTION

The present invention relates generally to a device for sealing and joining or cutting tissue. The device of the present invention is especially intended for use during either conventional open surgery or endoscopic or laparoscopic surgery.

### BACKGROUND OF THE INVENTION

Hemostasis, or blood clotting, can be obtained by the activation of a naturally occurring biological pathway known as the coagulation cascade. The pathway can be activated by tissue injury. This injury can come from mechanical, chemical or thermal sources. This natural biological pathway results in the conversion of freely flowing blood to a blood clot. Several biological elements are involved in the coagulation cascade, including tissue proteins, mainly fibrin and thrombin. Cells such as platelets and red and white blood cells are also involved.

During surgery, hemostasis can also be achieved by direct denaturization of the proteins found in the blood. Denaturization of a protein means that its characteristic three dimensional structure is altered without actually breaking up the protein. This direct denaturization is a purely physico-chemical process in which the denatured proteins bond together, forming an amorphous mass of protein which is comparable to a naturally occurring clot. How does denaturing a protein cause it to stick together with neighboring proteins? Proteins generally have a complex three-dimensional structure. A protein is actually a chain of smaller molecules called peptides, which peptides may have side-chains which contain a molecular group which can attract a molecular group on another side chain. The main protein chain is looped and folded on itself in a complex way which results in the three-dimensional structure characteristic of the protein. This looping and folding occurs because of an intra-molecular attraction between side-chains of the peptides. This attraction between side-chains is generally of the "hydrogen bond" or electrostatic type. The attraction which holds the peptides together along the main chain is a covalent bond. When a protein is denatured, it loses its normal three-dimensional structure. As a result of this unfolding of the protein molecule, the side-chains on the peptides, instead of facing "inward" to fold up the protein chain are now able to bond to side chains from proteins which are neighbors. This inter-molecular bonding results in the formation of large a lump of denatured protein. This process is not dependent on the activation of the biological cascades of the natural clotting mechanism, but it is a purely physico-chemical process. For hemostasis, the tissue proteins which must be denatured are chiefly those in blood such as hemoglobin and albumin but also include structural proteins such as those found in the wall of blood vessels or in other anatomical structures.

One of the best ways to denature a protein is to heat it up to a temperature high enough to cause the intra-molecular hydrogen bonds to break, but which is not high enough to break the much stronger peptide-peptide covalent bonds along the main chain. A prime example of this process is the heating up of the clear part of an egg until it turns white. This white color means that the original clear protein has been denatured.

Heat which is delivered to tissue proteins may start out as electrical energy, light energy, radiowave energy, or mechanical (vibrational or frictional) energy. As far as the tissue is concerned, it does not matter what the original source of the original energy is, as long as it gets converted in some fashion to heat.

For example, if the source of the energy is a laser, then the light energy is absorbed by molecules in the tissue whose absorption spectrum matches the wavelength of the laser light being used. Once the light energy is absorbed, heat is produced, and the physico-chemical process of protein denaturation is achieved. Any sort of light energy will have this effect, if its wavelength is such that it can be absorbed by the tissue. This general process is called photocoagulation. The advantage of using a laser is that since its output is monochromatic, one can selectively heat certain tissue elements which have the right absorption spectrum, while sparing other tissue elements for which the laser light is not absorbed. This principle is used commonly in ophthalmology. Another advantage of using a laser is that its coherent and collimated beam can be very tightly focused on very small targets. If one does not care about spatial precision or selective photocoagulation of only certain tissue elements, then it is perfectly possible to coagulate tissue by using a very bright but otherwise ordinary light.

If the source of energy is electrical currents flowing through the tissue, the process is called "electrosurgery". What happens here is that the current flowing through the tissue heats up the tissue because the tissue has resistance to the flow of electricity ("Ohmic heating"). In the case of ultrasonic coagulation, the rapid vibration of the ultrasonic element induces heating in essentially the same fashion as the production of fire by rubbing sticks together (although the rate of vibration is much much higher and the process is more controllable).

Since it is heat that denatures and coagulates proteins, why go to all the trouble of starting with a laser or an electrosurgery unit? Why not just use a very simple source of heat, such as a resistance wire or, even simpler, a hot piece of metal? In antiquity, "cautery" via a hot piece of iron was used to staunch bleeding wounds. The problem with this approach is not efficacy, it is control and containment of the amount and extent of tissue which is cauterized or injured.

In fact, the development of "electrocautery" in the late 1920's by Professor of Physics William T. Bovie was spurred by the desire (of the pioneering neurosurgeon Dr. Harvey Cushing) to have a more controllable and refined means of producing heat in tissues than possible by using a large piece of metal. Electrocautery uses very high frequency alternating electrical current, since it was found that these high frequencies did not cause tetanic ("Galvanic") stimulation of muscle tissue which occurs when direct current or low frequency current is used. To avoid muscular stimulation, it is necessary to use alternating currents with very high frequencies, about several hundred thousand cycles-per-second. This high frequency falls in the range of the AM radio band, which is the reason why many electrical devices such as monitors used in the OR will register interference when electrocautery is activated. There are many potential problems stemming from the use of such high frequencies, including difficulty in controlling stray currents which can injure patients and interfere with pacemakers and computer equipment. Electrocautery has been refined over the past fifty years, but it still represents a rather round-about way of getting tissue to heat up.

Numerous devices are known which coagulate, seal, join, or cut tissue. For example, there are electrosurgical devices, both monopolar and bipolar, which use high frequency electrical current that passes through the tissue to be coagulated. The current passing through the tissue causes the tissue to be heated, resulting in coagulation of tissue proteins. In the monopolar variety of these devices, the current leaves the electrode and after passing through the tissue, returns to the generator by means of a "ground plate" which is attached or connected to a distant part of the patient's body. In a bipolar version of such an electrosurgical device, the electric current passes between two electrodes with the tissue being placed or held between the two electrodes as in the "Kleppinger bipolar forceps" used for occlusion of Fallopian tubes.

There are many examples of such monopolar and bipolar devices commercially available today from companies including Valley Lab, Cabot, Meditron, Wolf, Storz and others worldwide. A new development in this area is the "Tripolar" device marketed by Cabot and Circon-ACMI which incorporates a mechanical cutting element in addition to monopolar coagulating electrodes.

With regard to known ultrasonic devices, a very high frequency (ultrasonic) vibrating element or rod is held in contact with the tissue. The rapid vibrations cause the proteins in the tissue to become coagulated. The ultrasonic device also employs a means for grasping the tissue while the proteins are being coagulated.

Olympus markets a heater probe device which uses an electrical heating wire contained in a catheter type flexible probe meant to be passed through a flexible endoscope. It is used to coagulate small bleeding vessels found on the inside of the gastrointestinal tract or the bleeding vessels found in peptic or other sorts of gastrointestinal ulcerations. In this device, no electrical current passes through the tissues, as is the case for monopolar or bipolar cautery. This device would certainly not be suitable for use in laparoscopic or open surgery in which large amounts tissue must be not only coagulated but also divided.

There are a number of known patents:
Pignolet, U.S. Patent No. 702,472, discloses a tissue clamping forceps with jaws wherein one has a resistance for heating the jaw, and a battery to power the heater. The coagulated tissue caused by the heat and pressure is subsequently severed along the edges of the jaws before they are opened;
Downes, U.S. Patent No. 728,883, teaches an electrothermic instrument having opposing jaw members and handle means for actuating the jaws. A resistance member is installed in the jaw member, which is closed to direct contact by a plate. This device coagulates tissue by heat, not electrical current, applied to the tissue;
Naylor, U.S. Patent No. 3,613,682, discloses a disposable battery-powered cautery device;
Hiltebrandt et al., U.S. Patent No. 4,031,898, concerns a coagulator with jaw members, one of which contains a resistance coil. This device has a timer mechanism for controlling the heating element. The heating element is used directly as a temperature sensor;
Harris, U.S. Patent No. 4,196,734, teaches a device that can effect both electrosurgery and cautery. A thermistor temperature-sensing element monitors a heating loop and regulates the current and thereby the temperature;
Staub, U.S. Patent No. 4,359,052, relates to a cautery device with removable, battery-powered cautery heating trip; Huffman, U.S. Patent No. 5,276,306, discloses a pistol-grip, hand-held heating device having a trigger mechanism for the battery;
Anderson, U.S. Patent No. 5,336,221, teaches an opt:ical thermal clamping device for welding or fusing tissue, and employing a cutting blade for separating the fused tissue;
Stern et al., U.S. Patent No. 5,443,463, discloses clamping jaw members that are bifurcated by a cutting blade, having plural electrodes and temperature sensors, and can function as monopolar or bipolar; and
Rydell, et al., U.S. Patent No. 5,445,638, relates to a bipolar coagulation and cutting instrument.
Andersen, U.S. Patent No. 5,300,065 discloses an apparatus for sealing approximated edges of tissue with thermal energy including a clamp having members which grasp and hold tissue and a laser energy source as a preferred energy source.

While each of the above mentioned references is relevant to the invention herein, none teaches or suggests the totality of the invention taught and claimed here.

### OBJECTS OF THE INVENTION

It is an object of the present invention to provide a device for sealing and cutting tissue.

It is also an object of the present invention to provide a device for sealing and joining tissue.

It is another object of the present invention to provide a portable device which does net require an external power source.

It is a further object of the present invention to provide a device which can be constructed to conform to the requirements of laparoscopic and endoscopic surgery, i.e., to be long and very narrow, in the range of a few millimeters in diameter or even narrower.

It is a still further object of the invention to provide an apparatus for optimal heating and optimal pressure to optimize tissue seal strength and to minimize collateral damage tc tissue.

These and other objects of the invention will become apparent to one skilled in the are from the following more detailed disclosure of the invention.

### SUMMARY OF THE INVENTION

The above objects are achieved by a device for sealing and/or cutting tissue according to claim 1.

According to the invention, there are three parameters that are independently controlled - the temperature to which the tissue is heated, the pressure which is applied, and the time over which the temperature and pressure are maintained. The total heat applied to the tissue is a function of the temperature and the time. A key feature is.the combined (simultaneous, partially simultaneous, or sequential) application of pressure and heat to the tissue being coagulated for a specified amount of time, which induces the denatured proteins to bond together, which in turn assists the attainment of hemostasis with less heat energy than would be required without the pressure. Also, the total energy applied is minimized by means of the configuration and materials of the parts of the device that hold the tissue in opposition during the application of the heat and pressure. Using less heat energy means less collateral damage. Also, results can be achieved that are at least as good as can be achieved with known electrosurgical and ultrasonic tissue coagulation units, but with a much smaller, lighter power source, such as a battery. Also, a very simple and direct method of heating the tissue is used. Since the basic heating element is so simple, the improved results can be achieved at a fraction of the cost of the more round-about means of heating tissue.

According to one aspect of the present invention a device for sealing, or coagulating, and cutting tissue during surgery are provided. The device incorporates means for controllably heating tissue while simultaneously applying a definite and controllable amount of pressure to the tissue being heated. Because of the combined application of heat and pressure, tissue proteins will become coagulated and blood vessels within the tissue will be sealed shut, achieving hemostasis. Optimal sealing or coagulating tissue means producing a strong and durable seal or coagulation or anastomosis with a minimal amount of collateral tissue damage. In the device of the invention optimization is achieved by a combination of the physical configuration of the part of the device that holds the tissue during the coagulation process and regulation of the time, temperature, and pressure.

As part of the control, the heat can be applied in pulses rather than in a continuous manner. Pulsed heat application allows tissue that is adjacent to the area being coagulated time to recover from the heating process and to remain viable. Also, the application of the pressure may be variable in intensity and may also be applied in a pulsed or discontinuous manner.

It is an aspect of the present invention to provide a device for the surgical treatment of biological tissue, wherein thermal energy and pressure are applied simultaneously, substantially simultaneously, consecutively, or alternatively, over a time such that tissue proteins are denatured and the tissue will adhere or join to itself or to other tissues, for the purpose coagulating bleeding, sealing tissue, joining tissue and cutting tissue. The minimum amount of heat or thermal energy needed to accomplish these goals is expended, so as to minimize thermal damage to tissue adjacent to the treated site.

The device may also incorporate means for cutting, or severing, the tissue after the tissue has been coagulated, "cutting" including dissecting or tissue division, tissue disruption or separation, plane development, or definition or mobilization of tissue structures in combination with a coagulation or hemostasis or sealing of blood vessels or other tissue structures such as lymphatics or tissue joining. The cutting can be achieved by means of a blade which is passed through the coagulated tissue while the tissue is being held in the jaws of the device. Cutting can also be achieved thermally by use of amounts of heat greater than the amount required to coagulate the tissues. In the case of using thermal energy to achieve tissue cutting, the device will use the minimum amount required to divide tissues with the least amount of unwanted tissue necrosis.

The heating element may be a resistance wire through which electric current is passed. The electrical current is applied through the wire either as a continuous current or as a series of pulses of definite duration and frequency. Unlike conventional electrosurgical devices, the electric current of the devices of the invention does not pass through the tissue, which can cause problems due to stray electric currents. The electrical elements are electrically insulated from the tissue while being in good thermal contact. In a simple embodiment of the device, the total amount of continuous current and hence the total heat energy applied to the tissue, is limited in duration by a simple timer circuit or even by direct visual or other sensory inspection of the treated tissue. In a more sophisticated embodiment, the pulse train configuration and duration is under control of a simple microcontroller, such as, for example, an embedded microprocessor. With microprocessor control, a thermistor heat sensor is incorporated into the part of the device that grasps the tissue being coagulated. The microprocessor takes temperature readings from the thermistor and adjusts the pulse train configuration and duration to achieve the optimum temperature to cauterize or seal the tissue while minimizing unwanted collateral thermal damage. The actual value of the optimum temperature can be verified experimentally for this particular device.

The temperature of the sealing treatment according to the invention is preferably kept in the range required to denaturate tissue proteins (approximately 45°C to below 100°C) while avoiding excessive necrosis to the tissue. Keeping the temperature in the range required to achieve protein denaturization without excessive tissue necrosis means that the total heat energy expended in the treatment will be less than if the temperature were not keep in this range. The amount of heat energy expended in the treatment is related to the degree of the heat (the temperature) and the length of time for which the heat is applied. The combined application of pressure with the heat reduces the amount of heat or the degree of temperature that would be required to have the denatured proteins actually stick together. This combined application of pressure also increases the strength with which the denatured proteins actually stick together, for a given amount of heat energy at a given temperature.

The amount of pressure applied is regulated by springs or other elastic elements, or mechanically functional equivalents, which will result in the tissue being held with a predetermined amount of force per unit area, in spite of variations in the size or thickness of the tissue being sealed or coagulated. The pressure may also be regulated by mechanical elements or spacers or by the geometry of the pressure producing elements. As with the temperature value, the exact value for the pressure to be applied can be verified for this device with appropriate measurement calibration.

The controlled application of a combination of heat and pressure which is sufficient but not excessive to produce a durable coagulation or seal has the result that only a relatively small amount of heat energy is needed. That only a relatively small amount of heat is needed means that relatively small electrical batteries can be used as the energy source to produce the heat. A device of the invention can therefore be free of bulky and heavy external power generators such as are required with conventional electrosurgical, laser or other devices for coagulating tissue. Because small batteries can be used to power the device, the device can be made quite compact and light weight, as well as portable and/or disposable. The use of batteries or other sources of low voltage direct current facilitates the avoidance of hazards and inconveniences caused by electrical interference and stray currents, which occur in conventional high-frequency elecgrosurgical devices. Laser eye hazards and also thereby avoided.

Since the heating elements and pressure producing elements of the device may be inherently simple and inexpensive to manufacture, the part of the device that comes in contact with tissue can be made in a disposable manner, if desired, while the more expensive portions of the device can be made to be reusable. If the device incorporates a simple timer, instead of the microprocessor-thermistor controller, the entire device including batteries can be made very inexpensively and to be disposable.

Different embodiments of this device employing the same general principle of controlled application of a combination of heat and pressure can be used to join or "weld" adjacent tissues to produce a junction of tissues or an anastomosis of tubular tissues. The joining of tissues is essentially a special case of the controlled coagulation of tissue proteins to achieve hemostasis.

It is a further aspect of the present invention that such effects will be spatially confined by the physical configuration and materials employed in the construction of the device. The configurations and construction materials are such that 1) the tissue is held in apposition with enough pressure to effect a strong union of the denatured proteins but not enough pressure to cause necrosis of the tissue, 2) the heat is concentrated on the tissue being treated by means of the material of the jaws which hold the tissue being treated, such material being a thermal insulator which prevents the heat from being expended on heating adjacent tissues. Such material may also employ a reflective layer or coating to reflect back the treated tissue heat energy that would otherwise be lost to thermal radiation. Such material may also have a geometry or be shaped in such a way to focus the thermal energy on the treated tissue and away from tissue not intended to be treated. For example, the jaws of the device may have a concave or parabolic inner surface to focus the thermal energy.

It is a further aspect of the present invention that such effects will be spatially confined by the kind, amount, and duration and temporal distribution of the energy delivery. The energy could originate as heat, light, sound or electricity, chemical, or other forms of energy, as long as this energy is converted to heat to denature tissue proteins. In a preferred embodiment, the energy would be delivered from a simple, low cost thermal heating element which could be powered by a battery contained in the device itself. The energy could be delivered in a continuous, or pulsed or intermittent mode, at variable or constant intensity. Pulsed or intermittent delivery of energy can produce a spatial confinement of the energy distribution. Feedback (including optical, thermal, spectroscopic, among others) and a microprocessor could be used to control the thermal effect. In the case of tissue coagulating, sealing or joining, the temperatures produced by the energy source would be the range of from about 45°C to about 100°C for a duration long enough to produce denaturation of the proteins in the treated tissue.

The heat or energy delivery source may be a simple electrically resistant wire, straight or curved, a grid or pattern of wires, or a thin-film or coating of electrically resistant material. One or more energy elements may be used. They may target some or all of the tissue treated by the pressure elements, The energy delivery source may be integral with or separate from the pressure elements. Cutting elements may be incorporated into the energy elements. The energy or heat source may move or be fixed. The energy may be delivered in a similar or dissimilar plane compared to the direction of pressure application. The energy or heat source may be constructed in such a way that its shape and size may be varied to conform to different anatomical situations, tissue shapes and thicknesses.

It is a further aspect of the present invention that such effects will be spatially confined by the kind, amount, and duration or temporal distribution of the pressure delivery acting in conjunction with the energy or heat source. The delivery of pressure will usually be from a minimum of two elements of the apparatus rather but may in some cases be from simple abutment or pressing of a single element against tissue, as in the example of the circular cutting wheel or a coring biopsy device. Any combination of geometric arrangement between the energy source and the pressure source may be produced, including combined energy-pressure sources and separate energy and pressure sources. A constant requirement is that the energy element deliver energy to at least some of the tissue that is subjected to pressure by the pressure element. The pressure element likewise may be variable in its shape, being able to adjust its shape before or during the application of the energy or pressure to accommodate for different anatomical situations, tissue shapes or thicknesses. Cutting elements or other elements for shaping or forming the tissue may be incorporated with the pressure element. For example, the pressure element may be comprised of a flattened side with an acute up-angled center to produce a combination of cutting effect over the center with compression along the sides. The pressure applied may be constant or variable over time and the relation of the pressure elements to the tissue may be constant or variable during application of the pressure and energy or both. Motion of the appropriately configured pressure elements may be used to effect cutting before, during or after application of the energy or pressure. The variable application may likewise be controlled by feedback from pressure transducers or strain sensors acting with a microprocessor.

It is an aspect of the invention that a completely separate cutting element could be used in addition to separate energy and pressure elements. It is also an aspect of the invention that mechanical tissue fastening devices including sutures, staples, clips, bands, screws, plates or tacks could be incorporated into the device. In this case the thermal energy and pressure would be used to provide mainly coagulation and sealing and the mechanical elements would provide additional strength to the tissue joint or anastomosis.

The invention can be used in either open, laparoscopic, endoscopic or any form of minimally invasive surgery. Surgical devices based on this invention could be long and thin, suitable for laparoscopic or minimally invasive approaches.

The parameters of temperature, time, pressure, as well as the any adjustable physical configuration or geometry of the device might vary depending on the type, size, and thickness of tissue being treated. These parameters may be experimentally determined before the actual treatment and incorporated into the device by means of a "look-up" table in a microprocessor or by means of simple markings and calibrations of adjustable knobs, dials, etc., of the device. An example useful for understanding the invention would incorporate two circular or cylindrical elements for the purpose of thermally joining or anastomosing two hollow tubular structures, e.g., small blood vessels or vas deferens. Such cylindrical elements would be designed to fit one into the other, acting as a jug or temporary stent which would hold the two tubular structures together while heat was applied. The tubular structures would be held in such a way to provide either a certain amount of overlap or end-to-end contact. As in previous embodiments, the amount of coaptive pressure which is being applied would be optimized according to the tissue type and thickness. The heat would be provided by a heating element or elements incorporated into the cylindrical jigs or stents and situated to apply the heat to the parts of the two tubular structures which are in overlap or in end-to-end contact. As discussed above, the amounts of heat and pressure applied are the minimum required to produce a secure anastomosis with the least amount of collateral damage.

Another example of this device would employ a circular mechanical cutting element, suitable for obtaining "core" biopsies of solid organs such as the liver or a kidney. This circular mechanical cutting element, shaped like a cylinder with sharp edges at one end, would incorporate an electrically resistant element on the outside of the cylinder. This electrically resistant element could be in the form of a thin film of resistance material. As the mechanical cutting of the tissue was done by rotating or pushing the cylindrical cutter into the tissue, hemostasis along the track created by the cutter would be achieved by the heating element on the outside of the cutter. The cylindrical cutter would be constructed out a material, or would incorporate a layer of a material, such that the tissue core sample being removed would be insulated from the thermal effects of the heating element on the outside of the core. This design would allow for retrieval of tissue samples which are not distorted by heat changes and also allow for secure hemostasis along the tract of the biopsy. In this device, the lateral pressure exerted by the cylinder wall on the tissues of the track cannot be explicitly controlled; however, there is pressure, and this pressure is part of the attainment of hemostasis.

In a further example useful for understanding the invention, a circular cutting wheel would be mechanically rotated to cut tissue, such as skin. This circular cutting wheel would incorporate along its rim, an electrically resistant thin film. This electrically resistant element would provide for hemostasis as the rotating mechanical wheel cuts the tissue.

In a yet further example, an inflatable elastic balloon could be used to apply heat and pressure to tissue. The exterior surface of the balloon would be coated partly or totally with flexible, optionally stretchable, electrically resistant material that will heat up when electrical current is applied. Here, the pressure exerted on the tissue can be controlled by regulation of the inflation pressure of the balloon.

Another embodiment of the invention is an improvement on endoscopic "hot biopsy" forceps such as that used for colonoscopy. Standard "hot biopsy" forceps use conventional electrosurgery power sources and unfortunately are associated with concerns over perforation of the bowel wall due to excessive cauterizing of the tissue of the bowel wall which is outside of the jaws of the forceps. Our embodiment of the "hot biopsy" forceps would incorporate an electrically resistant element only on the inner aspect of the biting part ("teeth") of the jaws of the forceps. The remainder of the jaw of the forceps would be made of a material which would provide thermal insulation to adjacent tissues. In this way a biopsy specimen could be obtained with good hemostasis and be protected from inadvertently cauterizing adjacent tissue outside the forceps, which could lead to perforation.

Yet another example useful for understanding the invention would be a snare-type device, similar in physical appearance and mechanical function to the polypectomy snares which are used during colonoscopy. Unlike conventional snares, which use monopolar electrosurgical power sources, the snare of this embodiment would use a specially configured wire or band which would have the electrically resistant material applied to the inner aspect of the wire or band for that portion of the wire or band which is used to make the actual loop which ensnares the tissue. This example would tend to direct the thermal energy into the substance of the tissue being grasped within the snare, as opposed to conventional electrosurgical snares which the electrical current can spread down the stalk or through the base of a polyp and cause damage to the underlying bowel wall and even perforation.

The device can be used in surgery and is particularly well suited to laparoscopic and endoscopic surgery. Because this method uses heat energy in the minimum amount and at the lowest temperature consistent with attaining denaturation and sticking together of tissue proteins, devices which work based on this method will be able to function more efficiently than conventional surgical energy devices. Therefore these devices can be portable and even battery powered, which makes them ideally suited for portable or military applications.

There is no device or method in the prior art which specifically seeks to obtain surgical coagulation, sealing, joining or cutting by a combination of heat energy and pressure at a time, temperature and pressure which together are sufficient but not excessive to produce protein denaturization, and with a physical configuration and materials of construction which promote the sticking together of the tissues being treated while minimizing losses of heat energy to surrounding tissues beyond the treatment zone.

### BRIEF DESCRIPTION OF THE DRAWINGS

Reference is made to the following description taken in connection with the accompanying drawings, in which:
Fig. 1 is a schematic representation of one embodiment of the present invention;
Fig. 1A is a cross-section along line I-I of the embodiment in Fig. 1 with the jaw in closed position;
Fig. 2 is a top, partly cross-sectional view of the lower jaw of the embodiment of Fig. 1 showing the heating and cutting elements;
Fig. 3 is a plan view of an example; Figs. 4 and 5 are cross-sectional views of the example of Fig. 3;
Figs. 6 and 6A are a plan view and a partial, enlarged view, respectively, of a further example ;
Figs. 7 and 7A are a plan view and a partial cross-sectional view, respectively, of another example;
Fig. 8 is a partly cross-sectional view of a further example;
Fig. 9 is a plan view of yet another example;
Fig. 10 is a top, partly cross-sectional view of the example of Fig. 9; and
Fig. 11 is a plan view of another example for heating and cauterizing tissue.

### DETAILED DESCRIPTION OF THE INVENTION

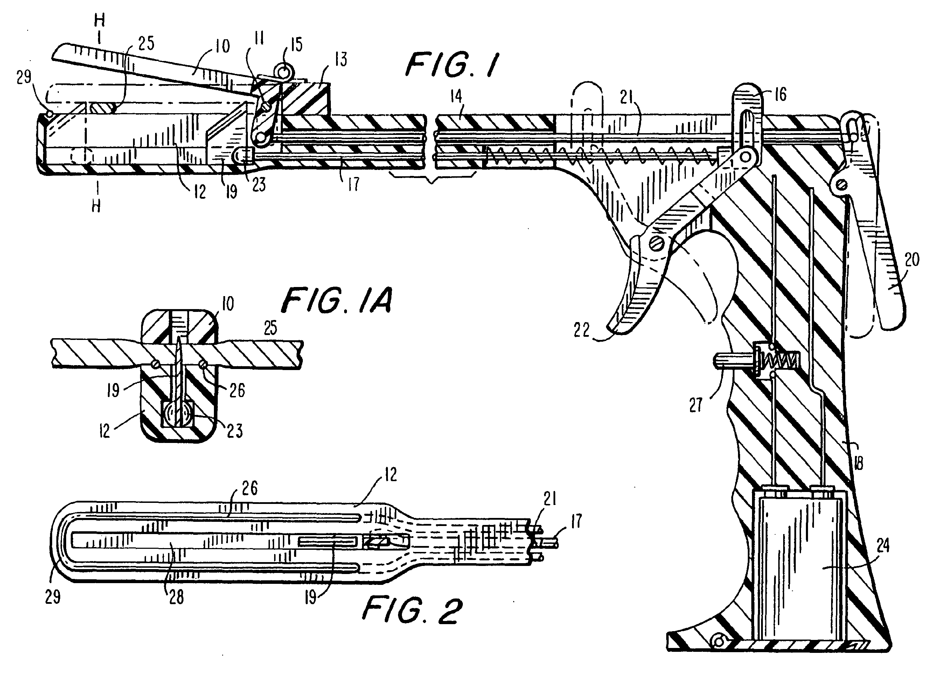

The invention can perhaps be better appreciated from the drawings. Fig. 1 depicts a schematic representation of the device of the invention showing an upper jaw 10, a lower jaw 12, an elongated shaft 14 attached to a handle 18, having a lever 20 for opening and closing the jaws. Upper jaw 10 is attached at hinge 11 to spring support member 13, and spring 15 is attached to both upper jaw 10 and spring support member 13 to bias upper jaw 10. Lever 20 is operatively connected through rod 21 to one or both of upper jaw 10 and lower jaw 12. The end of shaft 14 closest to handle 18 is provided with (1) a pusher 16 which is operatively connected through member 17 and connector 23 to a cutting knife blade 19 housed in lower jaw 12 and (2) a trigger 22 to actuate pusher 16 which in turn actuates cutting blade 19. The lower end of handle 18 is provided with a rechargeable battery pack 24, which is operatively connected to heating element actuator 27 and heating wire element 26 in lower jaw 12.

In Fig. 1A, tissue segment 25 is clamped between jaws 10,12, where it can be cut by blade 19.

Fig. 2 depicts a top view of lower jaw 12 showing the relative locations of heating wire element 26 and a slot 28 for cutting blade 19, within jaw 12. Heating wire element 26 is in a groove of a depth such that the wire is substantially flush with the surface of jaw 12. Preferably the distal portion 29 of heating wire element 26 is below, or out of, the plane of heating wire element 26 so that only two parallel areas of tissue will be sealed. Heating wire element 26, which preferably is comprised of nichrome or another suitable electrically resistant metal or alloy, or an electrically resistant thin-film or coating will preferably have a suitable, thermally conductive, electrically resistant, non-stick coating. Examples would include polytetrofluoroethylene (PTFE), e.g., TEFLON^{®}, or other non-stick coatings used in cookware. Moreover, one or both of the facing surfaces of upper jaw 10 and lower jaw 12 may optionally be corrugated, irregular, or grooved.

Both the upper and lower jaws are composed of a material, such as ceramic, which is thermally insulating or thermally reflective. In this way, the heat generated by the heating element is confined to the space between the jaws, and is not allowed to spread or radiate to other tissues that may be in contact with the outside of the jaws. This is beneficial in two ways: first, the heat generated by the heating element is used efficiently to perform the desired sealing or coagulation, and second, surrounding tissues are protected from inadvertent thermal injury.

As would be appreciated by one skilled in the art, the heating, pressure, and/or cutting functions could be mechanically, electromechanically, or electronically synchronized to obtain optimal results according to the invention. Also, the device shown in Figs. 1, 1A, and 2 may optionally not have a cutter element. Such a device would be intended for situations where only heating and pressure would be necessary to join tissue or to otherwise heat and cauterize tissue to produce coagulation.

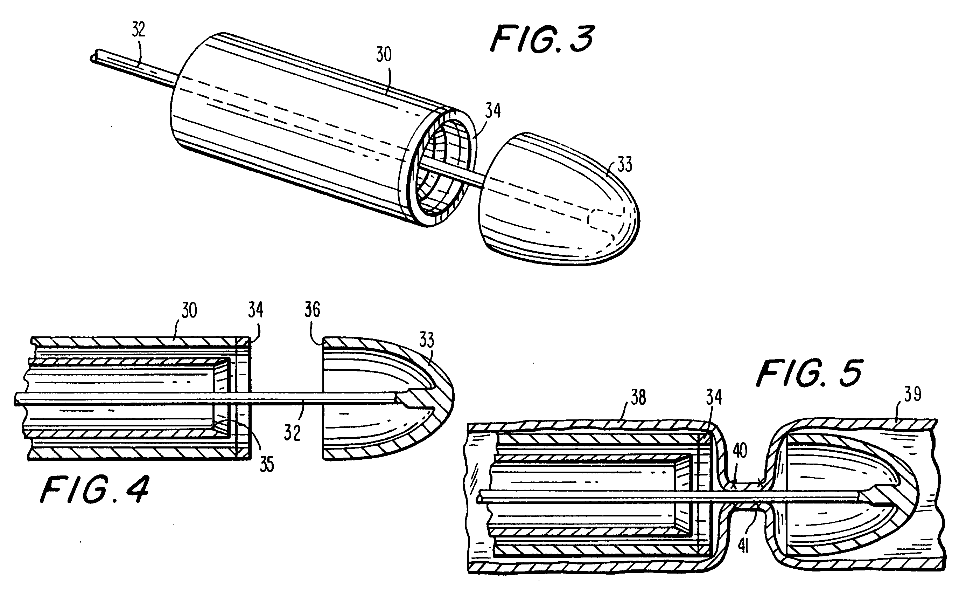

In the example useful for understanding the invention shown in Figs. 3 and 4, a cylindrical member 30 is concentrically positioned around a rod 32, the distal portion of which forms anvil 33. The distal surface of cylindrical member 30 comprises a circular heating element 34 and a circular cutting element 35 arranged concentrically within heating element 34. Anvil 33 is configured so that when rod 32 is moved proximally, the proximal circular edge 36 of anvil 33 cooperates with heating element 34 to coagulate or seal tissue.

Use of the example of Figs. 3 and 4 can be appreciated in Fig. 5, where, for example, two sections of intestine 38,39 are positioned to be joined together. Initially one end of each of sections 38,39 is loosely connected with ligatures 40,41 about rod 32. Then, rod 32 is moved distally to cause circular edge 36 of anvil 33 to force portions of intestines 38,39 into contact with heating element 34. Intestine sections 38,39 are joined together, and excess tissue is cut off by cutting element 35. Rod 32 is then pulled further in the proximal direction to remove the excess tissue, cylindrical member 30, and anvil 33.

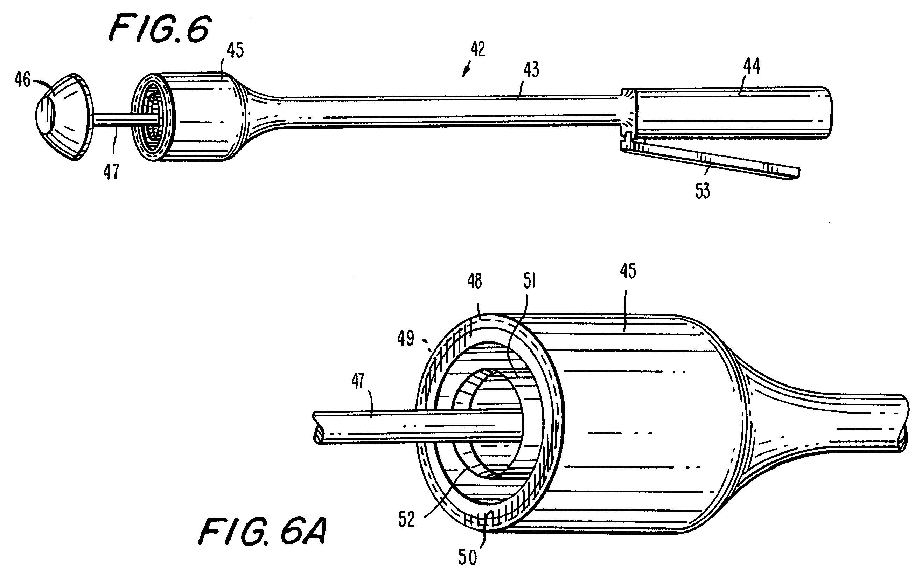

In addition, the device shown in Figs. 3 to 5 to produce circular anastomosis by relying on heat and pressure could additionally incorporate mechanical fastening elements such as staples. Such a device is shown in Figs. 6 and 6A, where a circular stapling device 42 comprises a main shaft 43, a handle 44, a staple housing 45, and an anvil 46. Anvil 46 is fixedly attached to the distal end of anvil shaft 47, which is movably slidable within staple housing 45, main shaft 43, and handle 44.

The distal surface 48 of staple housing 45 has slots 49 for staples (not shown) and an electrically resistant coating or member 50. An inner circular member 51 with a cutting edge 52 is arranged circumferentially around anvil shaft 47, as can be seen more clearly in Fig. 6A. Optionally, slots 49 and coating 50 could be coextensive.

Handle 44 comprises means for operating anvil 46 and heating element 49 and for firing the staples. As would be appreciated by those skilled in the art, a staple firing lever or member 53 can be operatively connected to a cylindrical pushing member within stapling housing 45 that causes the staples to be ejected from slots 49.

The operation of the circular stapling device would be similar to that of device shown in Fig. 3, with the exception that staples would be fired into tissue to be joined. Preferably the staples would be fired subsequent to sealing and concurrently with the cutting. The staples would act in conjunction with the thermal energy to enhance the strength of the tissue seal, joint or bond while the thermal energy would enhance the hemostatic capability of the staples. Staples or other mechanical tissue fasteners could be used in conjunction with thermal energy sealing in configurations other than circular, such as linear or angled.

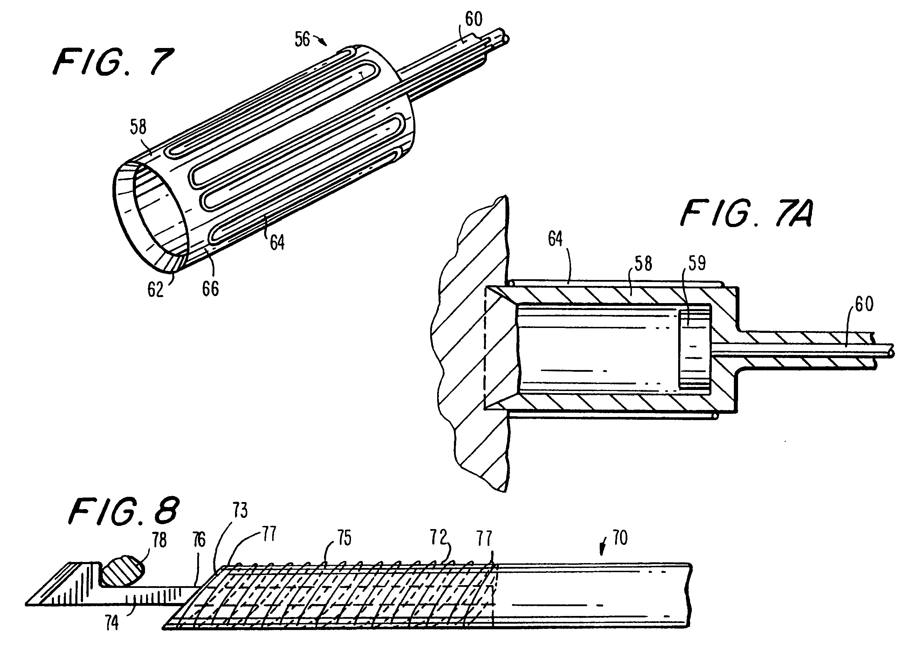

Fig. 7 depicts an example that is essentially a tissue-core removal device. The tissue-core removal device 56 comprises a cylindrical member 58 having a fixedly attached proximally extending handle 60. Cylindrical member 58 comprises a sharp cutting edge 62 and a heating element 64 arranged on the outer surface 66 of cylindrical member 58.

Consistent with the description above, a tissue sample is obtained by inserting removal device 56 into an organ, with device 56 being rotated as it moves forward. The rotation could be either clockwise or counterclockwise, but preferably alternatingly clockwise and counterclockwise, with sufficient pressure to cause edge 62 to cut. Heating element 64 will cauterize or seal tissue adjacent to the tissue sample to be removed, and when a tissue sample of sufficient depth is positioned within cylinder 58, device 56 will be removed. As is conventionally done, removal device 56 would preferably contain means for removing a tissue sample, such as an internal piston 59 having a proximally-extending actuator 60 to force the sample to be ejected from the distal end of removal device 56. As would be appreciated by those skilled in the art, a tissue-core removal device may optionally have additional cutting means at its distal end to assist in separation of a core tissue sample from the tissue mass.

In Fig. 8 the distal portion 70 of an electrothermal biopsy needle comprises an outer cutting sheath 72 slidably circumferentially arranged around an inner slotted stylus 74 having a slot 76 to capture a tissue sample 78. The outer sheath 72 has a cutting edge 73 which separates tissue sample 78 from the rest of the tissue mass (not shown) and encloses sample 78 in slot 76 when outer sheath 72 is propelled distally by an actuator (not shown).

Outer sheath 72 preferably has an electrically resistant film 75 coating on its distal portion. Film 75 may have spaced-apart electrical contacts or connectors 77. In another embodiment of a biopsy needle where stylus 74 has an inner cutting member (not shown), the stylus or the inner cutting member, or both, may have an electrically resistant coating or film.

The aforementioned aspect could be incorporated into known biopsy devices. See, for example, U.S. Patents Nos. 4,600,014 and 5,595,185.

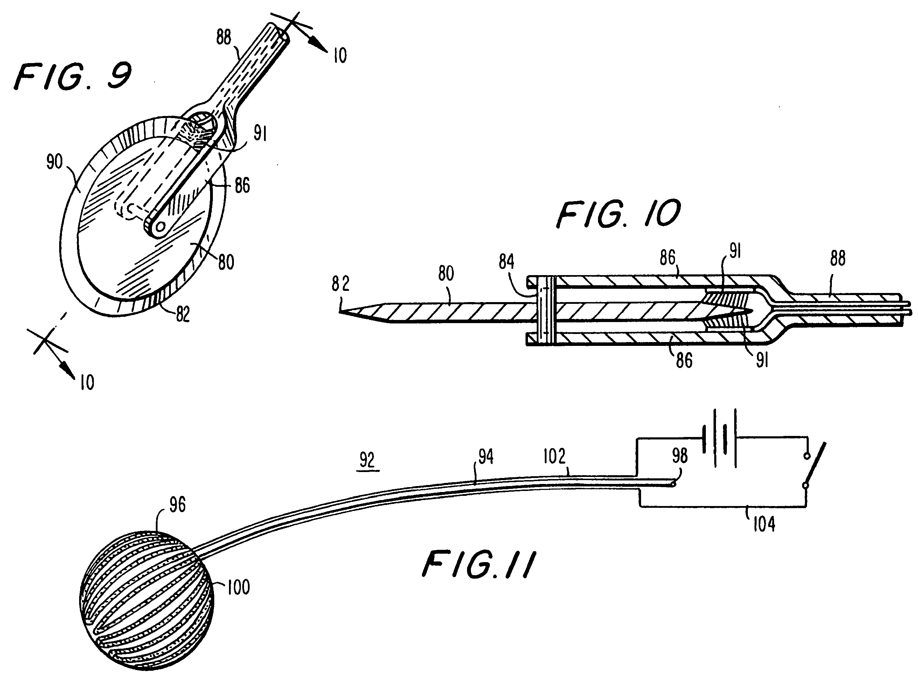

Figs. 9 and 10 depict a circular cutting device example in which a disk 80 having a sharp outer edge 82 is attached at its center to a rod 84 which is rotatingly secured to forks 86 of handle 88. Adjacent edge 82 is a circular heating element 90, which can be on one or both surfaces of disk 80. Each heating element 90 is electrically connected to fork 86, for example, through one or more brushes 91.

Fig. 11 represents an example where a heating and cauterizing device 92 comprises a catheter 94 and an inflatable balloon 96 sealingly attached to the distal end of catheter 94. Catheter 94 comprises at least one lumen 98, which is in fluid communication with balloon 96 for inflation and deflation. The proximal end of catheter 94 is in fluid communication with a regulated pressure source or inflation source (not shown) for inflating and deflating balloon 96.

Balloon 96 has an electrically resistant film coating 100, at least two separate portions of which are connected to wires 102 that extend proximally along or within catheter 94 to a power source 104. The electrically resistant film coating 100 is intended to cover a substantial portion, if not all, of the outer surface of balloon 96.

In use, device 92 with a deflated balloon 96 is manipulated within a patient's body, e.g., intracorporeally or even percutaneously, to position balloon 96 adjacent to a site to be cauterized. Then, balloon 96 is inflated so that the electrically resistant film coating 100 contacts the area to be cauterized, whereupon film coating 100 is energized with electrical energy from source 104. After the heat and pressure produce the desired effect, the power is turned off and the balloon is deflated to facilitate removal.

With regard to the examples depicted in Figs. 3 to 10, it should be appreciated that the respective heating elements are electrically connected to an appropriate power supply. It is envisioned that in each instance the power supply can be a battery or battery pack, which can be fixedly attached or integral with to the respective device. Optionally, the battery or battery pack could be separately mounted or positioned, such as on a clip or belt means for the operator to wear. It is within the scope of the invention that other standard sources of electrical power, such as transformers, may also be used. Other sources of heat such as fuel, e.g., butane, or chemical reactions, may be used.

As mentioned above, one aspect of the invention concerns optimization of (1) thermal energy application, i.e., temperature and time, and (2) pressure, i.e., force and duration, to achieve maximum tissue seal strength and minimal collateral tissue damage. Those skilled in the art will appreciate that useful parameters will vary greatly.

However, in practical application to human tissue a voltage of from about 0.5 volt to about 14 volts, preferably from about 1 volt to about 12 volts, will be applied to a heating element having a resistance sufficient to generate thermal energy to heat tissue to a temperature adequate to cause denaturation of proteins. This temperature is in the range of about 45°C to about 100°C. The pressure applied would be sufficient to provide coaptation but less than would crush or destroy the tissue itself.

The strength of tissue coagulations, seals, anastomoses or welds can be experimentally measured. For example, the strength of a coagulation produced on the side of a lacerated blood vessel can be measured experimentally by first producing the coagulation and then applying measured amounts of hydrostatic pressure to the inside of the vessel until the coagulation blows off and bleeding recommences. The strength of a tissue weld can be measured by first joining two pieces of tissue together and then placing the joined tissues in a machine which attempts to pull the tissue apart with increasing and measured amounts of force. Collateral thermal damage is also a measurable quantity in that the amount of collateral thermal damage can be readily assessed visually or microscopically. By use of this methodology, a table of optimized parameters could be constructed for any type of tissue. These parameters would be incorporated into the various devices by means of selecting the voltage, current, and resistance of the heating elements and also the amount of pressure used to press the tissue together during the coagulating/sealing/joining process, as well as the time duration of the process. These parameters can simply be incorporated into the device (i.e., simple mechanical timer, fixed preset voltage and current, and spring-loaded pressure devices, or, we can incorporate more flexible and active controls based on microprocessor regulation of the heating process, guided by a "look-up" table in ROM and by using sophisticated mechanical force/pressure sensors and strain gauges). Also, for certain applications, it may be sufficient to have a skilled operator, visually or by other sensing means, determine the duration of energy application and the amount of pressure required.

The devices of the present invention may be constructed of any suitable material, such as will be familiar to one skilled in the art, for example, out of a reinforced engineered plastic such as fiberglass reinforced polycarbonate, or machinable or injection-molded ceramics, or high temperature glass or epoxies, or mica. Alternatively they may be constructed out of a suitable alloy steel such as 318 stainless steel, or the like. The heating element may be a simple resistive wire or may be a thin film or coating composed of metallic, organo-metallic, or organic materials which may be conducting or semi-conducting. The actual materials of construction will be a matter of choice depending upon whether the device is to be employed repetitively or in a disposable manner. Indeed, in the latter situation it is contemplated that different parts of the device may be constructed of metal alloy and/or plastic, in which situation the plastic disposable components can be thrown out after each use and the more expensive metal alloy components reused. If sophisticated and expensive control circuitry is used, this part of the device could be made in a reusable manner.

It is intended that all matter contained in the above description and shown in the accompanying drawings shall be interpreted as illustrative and not in a limiting sense. Also, it is understood that the following claims are intended to cover all of the generic and specific features of the invention herein described and all statements of the scope of the invention which, as a matter of language, might be said to fall therebetween.

## Claims

1. A device for sealing and/or cutting tissue which comprises:
(a) an elongated member having distal and proximal ends, the proximal end of which is attached to a handle;
(b) two oppositely-positioned upper and lower jaw members each having proximal and distal ends, and each having a working surface, the jaw members positioned at the distal end of the elongated member and rotatably attached to each other at their respective proximal ends; and
(c) a heating element located in or on the working surface of at least one jaw member;
(d) **characterized in that** the heating element is an electrically resistant wire, a thin film or a coating of electrically resistant material through which electrical current is passed.

2. The device of claim 1, wherein the device includes a microcontroller to adjust a pulse train configuration and duration of the electrical current.

3. The device of claim 2, wherein the device includes a thermistor heat sensor.

4. The device of one of the preceding claims, wherein the device is adjusted to pass an electrical current through the electrically resistant wire as a constant current or as a series of pulses of definite duration and frequency.

5. The device of one of the preceding claims, wherein the electrically resistant wire is arranged in a U-shaped groove in the surface of the jaw member and projects out of the plane of the surface of the jaw member, wherein the distal portion of the heating wire element is below the plane of the surface of the jaw member.

6. The device of one claims 1 to 4, wherein the electrically resistant wire is a grid or pattern of wires.

7. The device of one of the preceding claims, wherein the heating element is adjustable to achieve a thermal cutting of tissue.

8. The device of one of the preceding claims, wherein the device comprises cutting means positioned in or adjacent to the working surface of a jaw member.

9. The device of Claim 8, wherein the cutting means comprises a blade and the blade is operatively connected to a cutting blade actuator.

10. The device of one of the preceding claims, wherein the device also comprises a battery pack attached to the handle and electrically connected to the heating element.

11. The device of one of the preceding claims, wherein a heating element actuator is operatively connected to the heater element.

12. The device of one of the preceding claims, which also comprises a jaw member activator operatively connected to at least one jaw member.

13. The device of one of the preceding claims which is disposable.

14. The device of one of the preceding claims, wherein the jaw member is fixedly attached to the elongated member.

15. The device of one of the preceding claims, wherein the jaw members are composed of a thermally insulating or thermally reflective material.

## Patentansprüche

1. Vorrichtung zum Verschließen und/oder Schneiden von Gewebe, welche umfaßt:
(a) ein gestrecktes Element mit einem distalen und proximalen Ende, wobei das proximale Ende an einem Griff befestigt ist;
(b) zwei gegenüberliegend positionierte obere und untere Klemmbackenelemente, wovon jedes ein proximales und ein distales Ende und jedes eine Arbeitsoberfläche aufweist, wobei die Klemmbackenelemente am distalen Ende des gestreckten Elements positioniert sind und an ihren jeweiligen proximalen Enden drehbar aneinander befestigt sind; und
(c) ein Heizelement, das in oder auf der Arbeitsoberfläche zumindest eines Klemmbackenelements angeordnet ist;
(d) **dadurch gekennzeichnet, daß** das Heizelement ein Draht mit elektrischem Widerstand, ein Dünnfilm oder eine Beschichtung aus einem Material mit elektrischem Widerstand ist, durch welches elektrischer Strom geführt wird.

2. Vorrichtung nach Anspruch 1, wobei die Vorrichtung einen Mikrokontroller umfaßt, um die Konfiguration einer Pulsreihe und Dauer des elektrischen Stroms einzustellen.

3. Vorrichtung nach Anspruch 2, wobei die Vorrichtung einen Thermistor-Wärmesensor umfaßt.

4. Vorrichtung nach einem der vorhergehenden Ansprüche, wobei die Vorrichtung dazu eingerichtet ist, einen elektrischen Strom durch den Draht mit elektrischem Widerstand als konstanten Strom oder als eine Reihe von Pulsen mit einer bestimmten Dauer und Frequenz zu leiten.

5. Vorrichtung nach einem der vorhergehenden Ansprüche, wobei der Draht mit elektrischem Widerstand in einer U-förmigen Nut in der Oberfläche des Klemmbackenelements angeordnet ist und aus der Ebene der Oberfläche des Klemmbackenelements vorragt, wobei sich der distale Abschnitt des Heizdrahtelements unter der Ebene der Oberfläche des Klemmbackenelements befindet.

6. Vorrichtung nach einem der Ansprüche 1 bis 4, wobei der Draht mit elektrischem Widerstand ein Netz oder ein Muster aus Drähten ist.

7. Vorrichtung nach einem der vorhergehenden Ansprüche, wobei das Heizelement einstellbar ist, um ein thermisches Schneiden von Gewebe zu erreichen.

8. Vorrichtung nach einem der vorhergehenden Ansprüche, wobei die Vorrichtung Schneidemittel umfaßt, die in oder in der Nähe der Arbeitsoberfläche eines Klemmbackenelements angeordnet sind.

9. Vorrichtung nach Anspruch 8, wobei das Schneidemittel eine Klinge umfaßt und die Klinge betriebstechnisch mit einem Schneidklingenbetätiger verbunden ist.

10. Vorrichtung nach einem der vorhergehenden Ansprüche, wobei die Vorrichtung auch ein Batterie-Paket umfaßt, das am Griff befestigt ist und elektrisch mit dem Heizelement verbunden ist.

11. Vorrichtung nach einem der vorhergehenden Ansprüche, wobei ein Heizelementbetätiger betriebstechnisch mit dem Heizelement verbunden ist.

12. Vorrichtung nach einem der vorhergehenden Ansprüche, welche ferner eine Klemmbackenelement-Aktivierungsvorrichtung umfaßt, die betriebstechnisch mit zumindest einem Klemmbackenelement verbunden ist.

13. Vorrichtung nach einem der vorhergehenden Ansprüche, welche ein Einwegartikel ist.

14. Vorrichtung nach einem der vorhergehenden Ansprüche, wobei das Klemmbackenelement fest am gestreckten Element befestigt ist.

15. Vorrichtung nach einem der vorhergehenden Ansprüche, wobei die Klemmbackenelemente aus einem thermisch isolierenden oder thermisch reflektierenden Material gebildet sind.

## Revendications

1. Dispositif permettant de sceller et/ou de couper des tissus comprenant :
(a) une pièce allongée avec des extrémités distale et proximale, dont l'extrémité proximale est fixée à une poignée ;
(b) deux pièces de mâchoires inférieure et supérieure agencées de façon opposée, chacune d'entre elle ayant des extrémités distale et proximale, ainsi qu'une surface active, les pièces de mâchoires étant positionnées au niveau de l'extrémité distale de la pièce allongée et fixées l'une à l'autre de façon rotative au niveau de leurs extrémités proximales respectives ; et
(c) un élément de chauffe situé dans ou sur la surface active d'au moins une pièce de mâchoire ;
(d) **caractérisé en ce que** l'élément de chauffe est un câble isolé, un film fin ou un revêtement d'un matériau isolé dans lequel un courant électrique passe.

2. Dispositif selon la revendication 1, dans lequel le dispositif comprend un microcontrôleur pour ajuster une configuration à train d'impulsion et la durée du courant électrique.

3. Dispositif selon la revendication 2, dans lequel le dispositif comprend un capteur de chaleur à thermistor.

4. Dispositif selon l'une des revendications précédentes, dans lequel le dispositif est ajusté de façon à faire passer un courant électrique dans le câble isolé de façon constante ou sous forme de séries d'impulsions d'une durée et d'une fréquence définies.

5. Dispositif selon l'une des revendications précédentes, dans lequel le câble isolé est agencé dans une rainure en forme de U ménagée sur la surface de la pièce de mâchoire et fait saillie par rapport au plan de la surface de la pièce de mâchoire, dans lequel la partie distale de l'élément de câble de chauffe est en dessous du plan de la surface de la pièce de mâchoire.

6. Dispositif selon l'une des revendications 1 à 4, dans lequel le câble isolé est une grille ou un réseau de câbles.

7. Dispositif selon l'une des revendications précédentes, dans lequel l'élément de chauffe peut être ajusté de façon à réaliser une coupe thermique des tissus.

8. Dispositif selon l'une des revendications précédentes, dans lequel le dispositif comprend des moyens de coupe agencés dans ou à côté de la surface active d'une pièce de mâchoire.

9. Dispositif selon la revendication 8, dans lequel les moyens de coupe comprennent une lame et la lame est connectée de façon opérative à un actionneur de lame de coupe.

10. Dispositif selon l'une des revendications précédentes, dans lequel le dispositif comprend également un bloc-pile fixé à la poignée et électriquement connecté à l'élément de chauffe.

11. Dispositif selon l'une des revendications précédentes, dans lequel un actionneur d'élément de chauffe est connecté de façon opérative à l'élément de chauffe.

12. Dispositif selon l'une des revendications précédentes, qui comprend en outre un actionneur de pièce de mâchoire connecté de façon opérative à au moins une pièce de mâchoire

13. Dispositif selon l'une des revendications précédentes qui est jetable.

14. Dispositif selon l'une des revendications précédentes, dans lequel la pièce de mâchoire est fixée de manière fixe à la pièce allongée.

15. Dispositif selon l'une des revendications précédentes, dans lequel les pièces de mâchoires sont composées d'un matériau isolant thermique ou à réfléchissement thermique.
